**Europäisches Patentamt**

**European Patent Office**

**Office européen des brevets**

(19)

(11) Numéro de publication: **0 253 743
B1**

(12)

# FASCICULE DE BREVET EUROPEEN

(45) Date de publication du fascicule du brevet:
**23.05.90**

(51) Int. Cl.⁵: **C07C 5/27,** B01J 29/12,
B01J 29/22

(21) Numéro de dépôt: **87401676.9**

(22) Date de dépôt: **16.07.87**

(54) Catalyseur d'isomérisation d'hydrocarbures, procédé de préparation et application dudit catalyseur.

(30) Priorité: **16.07.86 FR 8610345**

(43) Date de publication de la demande:
**20.01.88 Bulletin 88/3**

(45) Mention de la délivrance du brevet:
**23.05.90 Bulletin 90/21**

(84) Etats contractants désignés:
**AT BE DE FR GB IT NL SE**

(56) Documents cités:
**US-A- 4 057 489
US-A- 4 201 696
US-A- 4 252 686**

(73) Titulaire: **COMPAGNIE DE RAFFINAGE ET DE
DISTRIBUTION TOTAL FRANCE, 84, rue de Villiers,
F-92300 Levallois Perret(FR)**

(72) Inventeur: **Szabo, Georges, 38, rue de Normandie,
F-76290 Montivilliers(FR)**
Inventeur: **Cormerais, François-Xavier, 4, rue Guy de
Maupassant Fontaine la Mallet,
F-76290 Montivilliers(FR)**

(74) Mandataire: **Ohayon, Joseph et al, CABINET BROT ET
JOLLY 83, rue d' Amsterdam, F-75008 Paris(FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit
être formée par écrit et motivée. Elle n'est réputée formée qu'après paiement de la taxe d'opposition (Art. 99(1) Convention sur le
brevet européen).

## Description

La présente invention concerne des catalyseurs d'isomérisation d'hydrocarbures, notamment de normalparaffines de 4 à 7 atomes de carbone, un procédé de préparation desdits catalyseurs et l'application de ceuxci à l'isomérisation des hydrocarbures cités ci-dessus.

Pour isomériser des normal-paraffines, il existe deux types de procédés, à savoir des procédés à température relativement élevée, c'est-à-dire supérieure à 200°C, mettant en œuvre un catalyseur contenant un métal du Groupe VIII sur support silico-aluminate acide, et des procédés opérant à des températures plus basses, 150°C environ, mettant en œuvre un catalyseur de type Friedel-Crafts, comprenant par exemple le chlorure d'aluminium. Un procédé du premier type est décrit dans le brevet US-A 4 057 489 et un procédé du second type est décrit dans le brevet US-A 4 201 696.

Dans les procédés d'isomérisation, à température élevée, il est connu d'utiliser des catalyseurs à support zéolitique, dont les plus actifs et les plus sélectifs se composent de mordénite ou de faujasite seule ou en mélange avec un liant constitué d'un oxyde métallique réfractaire, comme l'alumine et la silice supportant un ou plusieurs métaux de la mine du platine.

Dans la suite de la présente demande, l'expression "métal de la mine du platine" désigne l'un des métaux suivants: le ruthénium, le rhodium, le palladium, l'osmium, l'iridium et le platine.

Parmi les catalyseurs bimétalliques sur support aluminosilicate ou mordénite, susceptibles de faciliter l'isomérisation du n-pentane en isopentane, les couples de métaux composés d'un métal de la mine du platine et d'un métal choisi dans le groupe constitué par le tungstène et le chrome sont préférés (voir brevet US 3 752 862).

Cependant, en étudiant ces catalyseurs à support zéolitique, la Demanderesse a observé que la présence de zirconium, en plus du platine et/ou de palladium sur la mordénite ou la faujasite en mélange avec de l'alumine, avait non seulement pour effet d'augmenter l'activité et la sélectivité de ces catalyseurs dans les procédés d'isomérisation, mais aussi de faciliter l'isomérisation des normal-paraffines à des températures et pressions plus faibles pour un taux de conversion plus élevé.

Le but de la présente invention est donc l'obtention de nouveaux catalyseurs à support zéolitique présentant des propriétés catalytiques (conversion et rendement en isoparaffines) satisfaisantes dans l'application à l'isomérisation des hydrocarbures.

Dans la suite de la présente description, le taux de conversion est défini comme étant la disparition relative d'une normal-paraffine et le rendement en isoparaffine est défini comme étant le rapport pondéral d'isoparaffine et de charge totale traitée.

La présente invention a par conséquent pour objet un catalyseur d'isomérisation de normal-paraffines en isoparaffine, caractérisé en ce qu'il comprend:

a) 49,9 % à 97 % en poids d'une zéolite choisie dans le groupe constitué par la mordénite et la faujasite, et de préférence 80 % à 97 % en poids de cette zéolite.

b) 0,1 à 1 % en poids de zirconium sous forme combinée.

c) 49,9 à 1 % en poids d'au moins un oxyde métallique réfractaire et de préférence 19,8 % à 1 % dudit oxyde, le préféré étant l'alumine.

d) et 0,1 à 1 % en poids d'au moins un métal choisi dans le groupe constitué par le platine et le palladium.

La teneur en zirconium du catalyseur selon l'invention est au moins égale à la teneur en platine et/ou en palladium et est de préférence comprise entre 0,4 et 0,7 % en poids du catalyseur.

En outre, la teneur en platine et/ou en palladium du catalyseur selon l'invention est de préférence comprise entre 0,2 et 0,4 % en poids du catalyseur.

Une autre caractéristique selon l'invention est que le zirconium et le platine et/ou palladium sont déposés sur des supports différents. Le zirconium est déposé sur une zéolite choisie dans le groupe constitué par la mordénite et la faujasite, après traitement chimique de ladite zéolite permettant de substituer une partie des ions alcalins présents par des ions hydrogène et d'extraire une partie de l'alumine présente dans le réseau zéolitique. Le palladium et/ou le platine est déposé sur le support zéolite - alumine, le zirconium ayant été déposé préalablement sur la zéolite.

La présente invention a pour autre objet un procédé de préparation de ces catalyseurs, caractérisé en ce qu'il consiste à :

a) traiter chimiquement la zéolite pour substituer au moins une partie des ions alcalins présents dans ce support par des ions hydrogène et pour extraire une partie de l'aluminium du réseau zéolitique,

b) déposer sur la zéolite le zirconium sous forme combinée,

c) mélanger l'alumine au support zéolitique sur lequel vient d'être déposé ledit métal,

d) calciner puis mettre en forme,

e) déposer le métal choisi dans le groupe constitué par le platine et le palladium,

f) sécher et calciner le catalyseur.

La mise en forme de l'étape d) peut être une extrusion ou tout autre procédé de mise en forme de poudre bien connu de l'homme de l'art.

EP 0 253 743 B1

Dans ce procédé de préparation, la substitution d'au moins une partie des ions alcalins contenus dans la zéolite par des ions hydrogène est connue par l'homme de l'art. Pour obtenir cette substitution, il a souvent recours à la technique dite des échanges d'ions en milieu acide ou dans une solution de sels. La désalumination partielle de la zéolite améliore souvent ses propriétés catalytiques. Elle est généralement effectuée, en plus de la technique des échanges d'ions, par traitement à la vapeur d'eau suivie d'une extraciton acide. Néanmoins, le taux de désalumination ne pourra excéder une certaine valeur au-delà de laquelle l'activité du catalyseur risque d'être pénalisée.

Dans la suite de la présente description, dans un souci de précision, on décrira plus en détail les solutions préférées pour effectuer le dépôt du zirconium, du platine et du palladium sur la zéolite et/ou l'alumine; toutefois, ceci ne doit pas être considéré comme une limitation de l'invention.

Dans le cas particulier du zirconium, on imprègne le support zéolitique par une solution aqueuse comprenant en mélange un sel de zirconium et son acide correspondant, ce sel étant choisi dans le groupe des halogénures, des oxalates et autres sels de zirconium ou de zirconyle.

Pour les cas du platine et du palladium, ils sont déposés par imprégnation du mélange zéolite-alumine avec une solution contenant un ion complexe du platine ou du palladium choisi dans le groupe constitué par les complexes du platine ou du palladium, en particulier les complexes aminés dont le platine dichloro tétramine et le palladium dichloro-tétramine. Pour une imprégnation homogène, il est en outre nécessaire d'introduire dans la solution un autre cation, de préférence le cation ammonium obtenu à partir d'un de ses sels, choisi dans le groupe constitué par les halogénures, le nitrate ou autres anions. Le cation ammonium et le platine et/ou le palladium présents dans la solution entrent en compétition dans la réaction d'échange avec les ions présents sur la zéolite ou l'alumine.

Un autre objet de l'invention peut être constitué par les catalyseurs préparés selon le procédé de préparation décrit ci-dessus.

L'invention consiste également en une application du catalyseur selon l'invention à l'isomérisation des normal-paraffines. Cette application peut être caractérisée en ce que, avant utilisation, ledit catalyseur peut être réduit dans une atmosphère sèche d'hydrogène sous une pression comprise entre 1 et 50 bars, de préférence entre 20 et 40 bars et à une température élevée comprise entre 200 et 600°C, de préférence 400 et 550°C.

L'invention a en outre pour objet un procédé d'isomérisation mettant en oeuvre ce catalyseur. Il peut être caractérisé en ce que l'isomérisation des normal-paraffines en isoparaffines est effectuée à une température comprise entre 220 et 280°C et de préférence entre 240 et 260°C, sous une pression totale comprise entre 2 et 40 bars et de préférence entre 5 et 30 bars, pour un rapport molaire hydrogène: hydrocarbure ($H_2$/HC) compris entre 0,5 et 4 et de préférence entre 1 et 2 et avec une vitesse spatiale déterminée par le poids de la charge passée par unité de poids de catalyseur par heure (pph) comprise entre 0,5 et 5 et de préférence entre 1 et 2.

Les résultats obtenus par isomérisation du n pentane au moyen de ce procédé sont très satisfaisants, comme on le verra dans le exemples donnés ci-après. On peut également isomériser le normal-butane, le normal-pentane, le normal-hexane, un mélange normal-pentane-normal-hexane, ou encore un mélange d'hydrocarbures paraffiniques de 4 à 7 atomes de carbone, enfin tout hydrocarbure paraffinique de température d'ébullition inférieure à 85°C.

Le catalyseur selon l'invention se distingue de celui du brevet US-A 4 057 489 mentionné précédemment par le fait qu'il en diffère:

— par sa composition puisqu'il comprend en plus de l'alumine et que le zirconium et le platine sont déposés sur des supports différents, respectivement la mordénite décationisée et le mélange mordénite — $Al_2O_3$,
— pour sa préparation puisque l'imprégnation de zirconium se produii en milieu acide et non en présence d'ammoniaque et que l'imprégnation de platine ne se fait pas en milieu acide comme pour l'imprégnation de palladium mais en présence de sel d'ammoniaque. De plus, le catalyseur selon l'invention n'est pas utilisé par la même réaction et sur les mêmes hydrocarbures que le catalyseur selon ledit brevet.

Le catalyseur selon l'invention peut être manipulé sans précaution particulière, alors que celui faisant l'objet du brevet US-A 4 201 696 mentionné précédemment ne doit l'être que sous atmosphère sèche contrôlée. De plus, les réactions d'isomérisation se produisent à des températures très différentes: 150°C environ pour le catalyseur selon ledit brevet et plus de 200°C pour celui selon la demande.

Les exemples donnés ci-après sont destinés à illustrer l'invention et n'impliquent aucune limitation de celle-ci.

EXEMPLE 1

Cet exemple décrit la préparation d'un catalyseur A et d'un catalyseur A' sans zirconium, obtenu à partir d'un mélange à 50% poids de mordénite et 50% poids d'alumine, sur lesquels ont été déposés 0,31% poids de platine (catalyseur A) et 0,2% poids de palladium (catalyseur A').

3

1) <u>Traitement chimique de la mordénite</u>

La mordénite utilisée est de l'alite 150 commercialisée par la Société Chimique de la Grande Paroisse, sous forme de poudre.

200 g d'alite 150 sont plongés dans deux litres d'acide chlorhydrique 6N; le mélange est maintenu à reflux pendant 6h30, puis la solution est filtrée et le filtrant est lavé à l'eau.

On effectue trois réactions d'échanges en plongeant ce filtrat dans une solution de nitrate d'ammonium 1M. Le rapport volumique liquide sur solide L/S de cette solution et du filtrat est égal à 10.

Ce mélange est maintenu sous reflux pendant une heure pour chaque échange. La solution contenant la mordénite trois fois échangée est filtrée.

Le filtrat recueilli, correspondant à la mordénite hydrogénée, dite mordénite $NH_4^+$ est lavé à l'eau distillée puis séché à 120°C pendant 18 heures.

Après calcination à 650°C on obtient une mordénite-H caractérisée par un rapport Si/Al égal à 8,7 et une teneur en oxyde de sodium ($Na_2O$) de 170 ppm.

2) <u>Mise en forme du mélange mordénite H - alumine</u>

12,25 g de mordénite H en poudre séchée à 120°C et 15,4 g de xérogel sont malaxés en présence de 32 cm³ d'eau tridistillée.

Le xérogel est préparé selon la technique bien connue par l'homme de l'art, c'est-à-dire en faisant réagir le trichlorure d'aluminium ($Al\ Cl_3$) ou le trinitrate d'aluminium ($Al(NO_3)_3$) avec une solution d'ammoniac ($NH_4OH$); le xérogel ($Al_2O_3$, $H_2O$) obtenu par cette réaction est filtré puis séché; il est exempt de sodium.

Le mélange mordénite-H-alumine malaxé est extrudé dans une filière de diamètre 2 mm. Les extrudés sont séchés une nuit à 120°C puis calcinés 2 h à 500°C après avoir démarré la chauffe dans un four à moufle froid.

3) <u>Dépôt de platine ou de palladium sur les extrudés mordénite-H-alumine</u>

On fait circuler sur 22,5 g d'extrudés une solution de nitrate d'ammonium ($NH_4NO_3$) 1 M pendant 2 heures, de telle façon que le rapport volumique liquide-solide $\frac{L}{S}$ de la solution et des extrudés soit égal à 5. Puis, pendant 24 heures, on fait circuler sur le solide une solution contenant au départ 14,9 g de nitrate d'ammonium ($NH_4NO_3$), 0,162 g de platine dichlorotétramine ($Cl_2Pt\ (NH_3)_4, H_2O$) et 300 cm³ d'eau tridistillée. Ce traitement permet au cation contenant le platine $[Pt(NH_3)_4]^{2+}$ de rentrer en compétition avec l'ion ammonium $NH_4^+$ dans la réaction d'échange avec les ions de la mordénite-$NH_4^+$.

Le catalyseur A ainsi obtenu est séché une nuit à 120°C et calciné pendant 3 h à 500°C.

Ce catalyseur ne contient que 0,07 % de chlore.

On obtient de façon comparable le catalyseur A', en utilisant 0,13 g de palladium dichlorotétramine ($Cl_2 Pd (NH_3)_4$ à la place du $[Cl_2 Pt (NH_3)_4, H_2O]$.

<u>EXEMPLE 2</u>

Cet exemple décrit la préparation d'un catalyseur B selon l'invention constitué d'un mélange 50-50 mordénite H-alumine, supportant 0,37 % de zirconium et 0,30 % de platine, et la préparation d'un catalyseur B' de même support contenant 0,35 % de zirconium et 0,20 % de palladium.

La mordénite H a été préparée comme décrit dans l'exemple 1.

1) <u>Dépôt du zirconium</u>

Une solution contenant 0,61 g de tétrachlorure de zirconium ($Zr\ Cl_4$) dans 150 cm³ d'acide chlorhydrique 1 N est introduite dans un évaporateur rotatif contenant déjà 30 g de mordénite H en poudre; le rapport volumique liquide-solide $\frac{L}{S}$ de la solution et de la mordénite H est égal à 5.

Cette solution est évaporée. Le solide obtenu est séché une nuit à 120°C puis calciné 2 heures à 500°C après avoir démarré la chauffe dans un four à moufle froid.

2) <u>Mise en forme du mélange (mordénite-H + Zr)-alumine</u>

30 g du solide calciné ci-dessus sont malaxés avec 42 g de xérogel en présence de 55 cm³ d'eau tridistillée. Le mélange malaxé est extrudé dans une filière de 2 mm. Les extrudés sont séchés une nuit à 120°C puis calcinés pendant 2 heures à 500°C après avoir démarré la chauffe dans un four à moufle froid.

### 3) Dépôt de platine ou de palladium

Ce dépôt est effectué comme décrit dans l'exemple 1; on fait circuler sur 31 g d'extrudés obtenus comme décrit ci-dessus une solution de nitrate d'ammonium ($NH_4NO_3$), 1 M pendant 2 heures, le rapport volumique liquide solide $\frac{L}{S}$ de la solution et des extrudés étant égal à 5.

Puis on fait circuler pendant 24 heures sur ces mêmes extrudés une solution constituée de 20,5 g de nitrate d'ammonium ($NH_4NO_3$), 0,23 g de ($Cl_2Pt$ ($NH_3$)$_4$, $H_2O$) et 410 cm³ d'eau distillée (pour le dépôt de palladium on utilise 0,18 g de $Cl_2Pd$ ($NH_3$)$_4$.

Les catalyseurs sous forme d'extrudés sont recueillis par filtration puis séchés pendant une nuit à 120°C. Ils sont calcinés pendant 3 h à 500°C.

La teneur en chlore mesurée est égale à 0,04 poids du catalyseur.

### EXEMPLE 3

Cet exemple décrit l'application des quatre catalyseurs A, A', B et B' préparés dans les exemples 1 et 2, à l'isomérisation de normal-pentane, de pureté voisine de 97, 5 %.

On introduit 20 g de catalyseur A ou A' ou B ou B' dans un réacteur de 42 cm³ d'un micro pilote d'isomérisation. Les catalyseurs sont conditionnés dans le réacteur par pressurisation du réacteur sous 30 bars d'hydrogène, avec régulation du débit d'hydrogène à 16,8 l/h, et augmentation de la température du réacteur jusqu'à 500°C à raison de 50°C/h, puis maintien de celle-ci 3 heures à 500°C.

La température du réacteur est alors diminuée jusqu'à 265°C, température à laquelle le normal-pentane est introduit dans le réacteur avec une vitesse spatiale ou pph de 2, pour un rapport hydrogène sur hydrocarbures $H_2$/HC égal à 1,25, sous une pression totale dans le réacteur égale à 30 bars.

Les résultats d'isomérisation obtenus dans ces conditions ainsi que la teneur en métaux des catalyseurs A et B sont rassemblés dans le tableau 1 suivant.

TABLEAU 1

| Catalyseur | Teneur en métal | | | Isomérisation | |
|---|---|---|---|---|---|
| | % Pd | % Pt | % Zr | % C | % RiC5 |
| A | | 0,31 | – | 59,7 | 59,7 |
| A' | 0,2 | | | 59,4 | 59,4 |
| B | | 0,30 | 0,37 | 63,8 | 63,8 |
| B' | 0,2 | | 0,35 | 63,7 | 63,7 |

% C = correspond au taux de conversion du normal-pentane

% RiC5 = correspond au rendement en isopentane de la réaction d'isomérisation

La présence du zirconium dans les catalyseurs B et B' comparativement aux catalyseurs A et A', pour une même teneur en platine ou en palladium favorise la conversion du normal-pentane en isopentane pour un craquage nul et, par conséquent, favorise l'augmentation du rendement de la réaction d'isomérisation.

### EXEMPLE 4

Cet exemple décrit l'isomérisation d'une charge industrielle avec un catalyseur C selon l'invention.

Le catalyseur C mis en oeuvre dans ce procédé est composé de 85 % en poids de mordénite - H et 15 % d'alumine supportant 0,26 % poids de platine et 0,63 % de zirconium par rapport au poids total du catalyseur. Il a été préparé de façon identique au catalyseur B, si ce n'est que les quantités de produits utilisées sont différentes.

20 g de ce catalyseur C ont été introduits dans le réacteur du micropilote d'une contenance de 42 cm³ et ont été conditionnés comme dans l'exemple 3 avant que la charge industrielle à isomériser soit introduite.

Cette charge est une essence légère de distillation dont l'indice d'octane (NOR) est de 70,6. Cette charge a la composition suivante :

| PRODUITS | % POIDS |
|---|---|
| $nC_4$ | 0,18 |
| $iC_5$ | 24,25 |
| $nC_5$ | 45,01 |
| $2,2dmeC_4$ | 0,42 |
| $CC_5$ | 1,19 |
| $2meC_5$ | 9,16 |
| $3meC_5$ | 4,88 |
| $nC_6$ | 11,76 |
| $meCC_5$ | 1,55 |
| $2,4dmeC_5$ | 0,11 |
| Benzène | 0,75 |
| $CC_6$ | 0,54 |
| $2meC_6$ | 0,09 |
| $2,3dmeC_5$ | 0,05 |
| $3meC_6$ | 0,06 |
| TOTAL | 100,00 |

Cette charge a été isomérisée dans différentes conditions selon le procédé d'isomérisation de l'invention. Ces conditions et les résultats de l'isomérisation de cette charge industrielle sont rassemblés dans le tableau 2.

TABLEAU 2

| T°C | Pression totale en bars | Vitesse spatiale (pph) | $H_2/HC$ Rapport molaire | CONVERSION | | Rdt + $C_5$ % pds | nombre d'octane de l'isomérat (NOR) |
|---|---|---|---|---|---|---|---|
| | | | | $nC_5$ | $nC_6$ | | |
| 260 | 32 | 2 | 2 | 38,8 | 55,7 | 99,0 | 81,4 |
| 250 | 30 | 1,5 | 1,5 | 46,7 | 62,48 | 98,9 | 83,3 |
| 240 | 12 | 1,0 | 2 | 45,1 | 62,2 | 99,0 | 82,9 |

Au vu de ce tableau, il apparaît que le procédé d'isomérisation mettant en oeuvre un catalyseur selon l'invention permet d'obtenir une essence à haut indice d'octane, environ 83, et ceci avec un excellent rendement en isoparaffines.

## EXEMPLE 5

Cet exemple compare les performances du catalyseur C décrit dans l'exemple 4 avec un catalyseur industriel X comprenant un support mordénite alumine et du platine. Ces deux catalyseurs sont appliqués à l'isomérisation du n-pentane.

On opère comme décrit dans l'exemple 4 pour le catalyseur C (20 g de catalyseur C dans le micropilote de 42 cm³). Pour le catalyseur X on charge également 20 g dans le micropilote, mais le volume occupé n'est que de 30 cm³. De cette façon, on opère à pph identique selon le procédé d'isomérisation de l'invention (les densités de chargement des deux catalyseurs sont différentes).

TABLEAU 3

| T°C | $H_2/HC$ Rapport molaire | Pression en bars | Vitesse spatiale pph | Catalyseur 3 | | Catalyseur X | |
|---|---|---|---|---|---|---|---|
| | | | | Rdt $C_5^+$ % pds | NOR | Rdt $C_5^+$ % pds | NOR |
| 250°C | 2 | 18 | 1,0 | 97,8 | 82,9 | 99,3 | 80,8 |
| 240°C | 2 | 12 | 1,0 | 98,0 | 82,9 | 99,5 | 80,0 |

EP 0 253 743 B1

On constate d'après le tableau ci-dessus que le catalyseur C selon l'invention est plus actif que le catalyseur X commercial en ce que l'indice d'octane (NOR) de l'essence obtenue dans les mêmes conditions avec le catalyseur C est toujours plus élevé que celui de l'essence obtenue avec le catalyseur X.

## Revendications

1. Catalyseur d'isomérisation de normal-paraffines en isoparaffines, caractérisé en ce qu'il comprend:
a) 49,9 % à 97 % en poids d'une zéolite choisie dans le groupe constitué par la mordénite et la faujasite et de préférence 80 % à 97 % en poids,
b) 0,1 à 1 % en poids de zirconium sous forme combinée,
c) 49,9 à 1 % en poids d'au moins un oxyde métallique réfractaire et de préférence d'alumine, et de préférence 19,8 % à 1 % dudit oxyde,
d) 0,1 à 1 % en poids d'au moins un métal choisi dans le groupe constitué par le platine et le palladium.

2. Catalyseur selon la revendication 1, caractérisé en ce que la teneur en zirconium dudit catalyseur est au moins égale à la teneur en platine et/ou palladium et est de préférence comprise entre 0,2 à 0,7 % en poids.

3. Catalyseur selon l'une des revendications 1 et 2, caractérisé en ce que la teneur en métal choisi dans le groupe constitué par le platine et le palladium dans ledit catalyseur est de préférence 0,2 à 0,4 % en poids.

4. Catalyseur selon l'une des revendications 1 à 3, caractérisé en ce que le zirconium est déposé sur une zéolite choisie dans le groupe constitué par la mordénite et la faujasite et de préférence après traitement chimique de ladite zéolite permettant de substituer une partie des ions alcalins présents par des ions hydrogène et pour extraire une partie de l'aluminium présent dans le réseau zéolitique.

5. Catalyseur selon l'une des revendications 1 à 4, caractérisé en ce que le métal choisi dans le groupe constitué par le palladium et le platine est déposé sur le mélange zéolite-zirconium-alumine.

6. Procédé de préparation du catalyseur défini par l'une des revendications 1 à 5, caractérisé en ce qu'il consiste à traiter chimiquement la zéolite choisie dans le groupe constitué par la mordénite et la faujasite pour substituer au moins une partie des ions alcalins présents dans ce support par des ions hydrogène et extraire une partie de l'aluminium présent dans le réseau zéolitique, à déposer le zirconium, à mélanger l'alumine audit support, à calciner puis à mettre en forme le mélange obtenu, à déposer le métal choisi dans le groupe constitué par le platine et le palladium, à sécher et à calciner ledit catalyseur.

7. Procédé selon la revendication 6, caractérisé en ce que le dépôt du zirconium est effectué par imprégnation dudit support d'une solution contenant un sel dudit métal choisi dans le groupe constitué par les halogénures, les oxalates et tous autres sels de zirconium et de zirconyle et l'acide correspondant au sel.

8. Procédé selon l'une des revendications 6 et 7, caractérisé en ce que le dépôt d'un métal choisi dans le groupe constitué par le platine et le palladium est effectué par imprégnation du support avec une solution contenant un ion complexe dudit métal choisi dans le groupe constitué par les complexes du platine et du palladium, en particulier les complexes aminés du platine dont le platine dichlorotétramine et le palladium dichlorotétramine et un sel d'ammonium en excès choisi dans le groupe constitué par les halogénures, nitrate et tout autre sel d'ammonium.

9. Catalyseur préparé par le procédé defini par l'une des revendications 7 et 8.

10. Application du catalyseur défini par la revendication 9 à l'isomérisation des normal-paraffines, caractérisée en ce que ledit catalyseur est réduit sous une atmosphère sèche d'hydrogène avec une pression comprise entre 1 et 50 bars, de préférence entre 20 et 40 bars, et à une température élevée comprise entre 200 et 600°C et de préférence entre 400 et 550°C.

11. Procédé d'isomérisation mettant en oeuvre le catalyseur selon l'une des revendications 9 et 10, caractérisé en ce que l'isomérisation des normal-paraffines est effectuée à une température comprise entre 220 et 280°C, de préférence entre 240 et 260°C, sous une pression totale comprise entre 2 et 40 bars, de préférence entre 5 et 30 bars, pour un rapport molaire $H_2/HC$ compris entre 0,5 et 4 et de préférence 1 et 2, et avec une vitesse spatiale exprimée en pph comprise entre 0,5 et 5, de préférence entre 1 et 2.

12. Application du procédé défini par la revendication 13 à l'isomérisation en isoparaffine du normal-pentane, du normal-hexane, du mélange normal-pentane-normal-hexane, du mélange d'hydrocarbures paraffiniques de 4 à 7 atomes de carbone et du normal butane.

## Patentansprüche

1. Katalysator zum Isomerisieren von normal-Paraffinen in iso-Paraffine, dadurch gekennzeichnet, daß er
a) 49,9 bis 97 Gew.-%, insbesondere 80 bis 97 Gew.-%, eines Zeoliths, gewählt aus der Gruppe bestehend aus Mordenit, Faujasit enthält,
b) 0,1 bis 1 Gew.-% Zirkonium in gekoppelter Form enthält,
c) 49,9 bis 1 Gew.-% wenigstens eines feuerfesten Metalloxides, vorzugsweise Aluminiumoxid, und insbesondere 19,8 bis 1 Gew.-% dieses Oxids enthält,

7

d) 0,1 bis 1 Gew.-% von wenigstens einem Metall, gewählt aus der Gruppe bestehend aus Platin und Palladium enthält.

2. Katalysator nach Anspruch 1, dadurch gekennzeichnet, daß der Zirkoniumgehalt des Katalysators wenigstens gleich dem Gehalt des Metalls, gewählt aus der Gruppe bestehend aus Platin und/oder Palladium ist und daß er vorzugsweise zwischen 0,2 bis 0,7 Gew.-% liegt.

3. Katalysator nach Anspruch 1 oder 2, dadurch gekennzeichnet, daß der Gehalt des Metalls, gewählt aus der Gruppe bestehend aus Platin und Palladium in dem Katalysator, vorzugsweise zwischen 0,2 und 0,4 Gew.-%, liegt.

4. Katalysator nach einem der Ansprüche 1 bis 3, dadurch gekennzeichnet, daß das Zirkonium auf einem Zeolith, gewählt aus der Gruppe bestehend aus Mordenit und Faujasit und vorzugsweise nach chemischer Behandlung des Zeoliths, welche es ermöglicht, einen Teil der vorhandenen Alkalimetallionen durch Wasserstoffionen zu ersetzen und einen Teil des in dem Zeolithgefüge enthaltenen Aluminiums zu extrahieren, abgeschieden wird.

5. Katalysator nach einem der Ansprüche 1 bis 4, dadurch gekennzeichnet, daß das Metall, gewählt aus der Gruppe bestehend aus Palladium und Platin auf der Mischung Zeolith-Zirkonium-Aluminiumoxid abgeschieden wird.

6. Verfahren zur Herstellung eines Katalysators nach einem der Ansprüche 1 bis 5, dadurch gekennzeichnet, daß das Verfahren in der chemischen Behandlung des Zeoliths, gewählt aus der Gruppe bestehend aus Mordenit und Faujasit besteht, um wenigstens einen Teil der in diesem Träger vorhandenen Alkalimetallionen durch Wasserstoffionen zu ersetzen und einen Teil des in dem Zeolithgefüge enthaltenen Aluminiums zu extrahieren, das Zirkonium abzuscheiden, das Aluminiumoxid dem Träer beizumischen, zu glühen und schließlich die erhaltene Mischung formen, das Metall, gewählt aus der Gruppe bestehend aus Platin und Palladium abzuscheiden, zu trocknen und den Katalysator zu glühen.

7. Verfahren nach Anspruch 6, dadurch gekennzeichnet, daß die Abscheidung von Zirkonium durch Imprägnierung des Trägers mit einer Lösung, welche ein Salz des Metalls, gewählt aus den Halogeniden, Oxalaten und allen anderen Zirkoniumsalzen und den Zirkonylen und der dem Salz entsprechenden Säure enthält, erfolgt.

8. Verfahren nach Anspruch 6 oder 7, dadurch gekennzeichnet, daß die Abscheidung des Metalls, gewählt aus der Gruppe bestehend aus Platin und Palladium durch Imprägnieren des Trägers mit einer Lösung, welche ein Komplexion des Metalls, gewählt aus der Gruppe bestehend aus den Platin- und Palladiumkomplexen, insbesondere den Platinaminkomplexen, wie Dichlortetraminplatin und Dichlortetraminpalladium und ein Ammoniumsalz im Überschuß, gewählt aus der Gruppe bestehend aus den Halogeniden, Nitraten und allen anderen Ammoniumsalzen enthält, erfolgt.

9. Katalysator, welcher mit den Verfahren nach einem der Ansprüche 7 oder 8 hergestellt wurde.

10. Verwendung des Katalysators nach Anspruch 9 zur Isomerisierung von normal-Paraffinen, dadurch gekennzeichnet, daß der Katalysator in einer trockenen Wasserstoffatmosphäre bei einem Druck zwischen 1 und 50 bar, vorzugsweise zwischen 20 und 40 bar bei einer Temperatur zwischen 200 und 600°C, vorzugsweise zwischen 400 und 500°C, reduziert wird.

11. Isomerisierungsverfahren unter Verwendung des Katalysators nach einem der Ansprüche 9 und 10, dadurch gekennzeichnet, daß die Isomerisierung der normal-Paraffine bei einer Temperatur zwischen 220 und 280°C, vorzugsweise zwischen 240 und 280°C, bei einem Gesamtdruck zwischen 2 und 40 bar, vorzugsweise zwischen 5 und 30 bar, mit einem Molverhältnis $H_2/HC$ zwischen 0,5 und 4, vorzugsweise zwischen 1 und 2, und mit einer Raumgeschwindigkeit, ausgedrückt in pph, zwischen 0,5 und 5, vorzugsweise zwischen 1 und 2, durchgeführt wird.

12. Verwendung des Verfahrens nach Anspruch 11 zur Isomerisierung von normal-Pentan, normal-Hexan, einer Mischung von normal-Pentan und normal-Hexan, Mischungen von Paraffin-Kohlenwasserstoffen mit 4 bis 7 C-Atomen und normal-Butan in Iso-Paraffine.

## Claims

1. A catalyst for isomerising normal paraffins into isoparaffins, characterised in that it comprises:
a) 49.9% to 97% of a zeolite selected from the group comprising mordenite and faujasite and, preferably, 80 to 97% by weight;
b) 0.1 to 1% by weight of zirconium in combined form;
c) 49.9% to 1% by weight of at least one refractory metal oxide and, preferably, alumina and, preferably, 19.8% to 1% of said oxide;
d) 0.1 to 1% by weight of at least one metal selected from the group comprising platinum and palladium.

2. A catalyst according to claim 1, characterised in that the zirconium content of said catalyst is at least equal to the platinum and/or palladium content and, preferably, is between 0.2 to 0.7% by weight.

3. A catalyst according to either of claims 1 and 2, characterised in that the content of the metals selected from the group comprising and palladium in said catalyst is preferably 0.2 to 0.4% by weight.

4. A catalyst according to any one of claims 1 to 3, characterised in that the zirconium is deposited on a zeolite selected from the group comprising mordenite and faujasite, preferably after chemical treatment of said zeolite enabling some of the alkaline ions present to be substituted with hydrogen ions and to extract some of the aluminium present in the zeolitic network.

5. A catalyst according to any one of claims 1 to 4, characterised in that the metal selected from the group comprising platinum and palladium is deposited on the zeolite-zirconium-alumina mixture.

6. A method of preparing the catalyst defined in one of claims 1 to 5, characterised in that it comprises chemically treating the zeolite selected from the group comprising mordenite and faujasite so as to substitute at least some of the alkaline ions present in this support with hydrogen ions and extract some of the aluminium present in the zeolitic network, then depositing the zirconium, mixing the alumina with said support, calcining and then shaping the reslutant mixture, depositing the metal selected from the group comprising platinum and palladium, drying and calcining said catalyst.

7. A method according to claim 6, characterised in that the deposition of zirconium is carried out by impregnating said support with a solution containing a salt of said metal selected from the group comprising the halides, oxalates and all other salts of zirconium and zirconyl and the acid corresponding to the salt.

8. A method according to either of claims 6 and 7, characterised in taht the deposition of a metal selected from the group comprising platinum and palladium is carried out by impregnating the support with a solution containing a complex ion of said metal selected from the group comprising the complexes of platinum and palladium, in particular the amino complexes of platinum such as platinum dichlorotetramine, and palladium dichlorotetramine, and an excess of an ammonium salt selected from the group comprising the halides, nitrate and any other ammonium salt.

9. A catalyst prepared by the method defined in either of claims 7 and 8.

10. Application of the catalyst defined in claim 9 to the isomerisation of normal paraffins, characterised in that said catalyst is reduced in a dry hydrogen atmosphere at a pressure of between 1 and 50 bar, preferably between 20 and 40 bar, and at a high temperature of between 200 and 600°C and, preferably, between 400 and 550°C.

11. An isomerisation method utilising the catalyst according to either of claims 9 and 10, characterised in that the isomerisation of normal paraffins is carried out at a temperature of between 220 and 280°C, preferably between 240 and 260°C, at a total pressure of between 2 and 40 bar, preferably between 5 and 30 bar, for a molar ratio H2/HC of betwen 0.5 and 4 and, preferably between 1 and 2, and with a spatial velocity expressed in pph of between 0.5 and 5, preferably between 1 and 2.

12. Application of the method defined in claim 13 to the isomerisation into isoparaffin of n-pentane, of n-hexane, of a mixture of n-pentane and n-hexane, of a mixture of paraffinic hydrocarbons having 4 to 7 carbon atoms and n-butane.